(19) Europäisches Patentamt
European Patent Office
Office européen des brevets

(11) Veröffentlichungsnummer: **0 094 657 B1**

# (12) EUROPÄISCHE PATENTSCHRIFT

(45) Veröffentlichungstag der Patentschrift: **26.08.87**

(51) Int. Cl.⁴: **C 07 D 493/10** // B41M5/16, B41M5/18 ,(C07D493/10, 311:00,307:00)

(21) Anmeldenummer: **83104776.6**

(22) Anmeldetag: **14.05.83**

## (54) Fluoranverbindungen, Verfahren zu ihrer Herstellung und ihre Verwendung als Farbbildner in Kopiersystemen.

(30) Priorität: **18.05.82 DE 3218645**

(43) Veröffentlichungstag der Anmeldung: **23.11.83 Patentblatt 83/47**

(45) Bekanntmachung des Hinweises auf die Patenterteilung: **26.08.87 Patentblatt 87/35**

(84) Benannte Vertragsstaaten: **BE CH DE FR GB LI**

(56) Entgegenhaltungen:
**DE - A - 2 121 269**
**DE - A - 3 225 028**
**GB - A - 1 478 596**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Bingmann, Horst, Dr., Zu den Eichen 13, D-6238 Hofheim am Taunus (DE)**

**EP 0 094 657 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung sind neue Fluoranverbindungen der allgemeinen Formel (1)

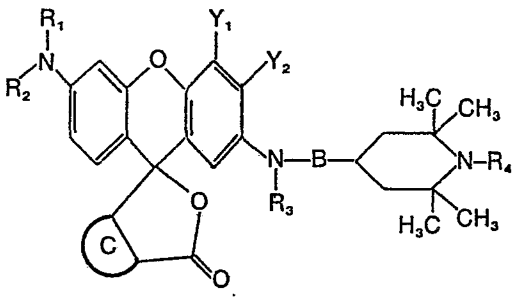

(1)

in welcher $R_1$ und $R_2$ unabhängig voneinander jeweils ein Wasserstoffatom oder eine Alkyl-$C_1$–$C_4$- oder Benzylgruppe, $R_3$ und $R_4$ unabhängig voneinander jeweils ein Wasserstoffatom, eine Alkyl-$C_1$–$C_4$-, Benzyl-, Acetyl-, Propionyl- oder Benzoylgruppe, $Y_1$ und $Y_2$ unabhängig voneinander jeweils ein Wasserstoff-, Chlor- oder Bromatom, Alkyl-$C_1$–$C_4$-, Alkoxy-$C_1$–$C_4$-, Phenyl- oder Naphthylgruppe, B eine direkte Bindung oder die 1,2-Ethylengruppe, und C den ggfs. durch Chlor- oder Bromatome substituierten 1,2-Phenylenrest bedeuten, ferner Verfahren zu ihrer Herstellung sowie ihre Verwendung als Farbbildner in einem druck- oder wärmeempfindlichen Aufzeichnungsmaterial.

Die Herstellung der neuen Fluoranverbindungen der genannten Formel (1) kann nach an sich bekannten Verfahren erfolgen. So können diese hergestellt werden, indem man eine Carbonsäure der allgemeinen Formel (2)

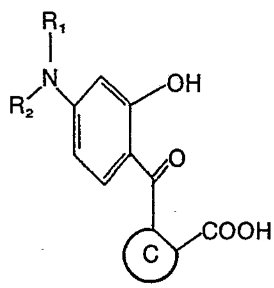

(2)

in welcher $R_1$, $R_2$ und C die weiter oben genannten Bedeutungen haben, mit einer p-Aminophenolverbindung der allgemeinen Formel (3)

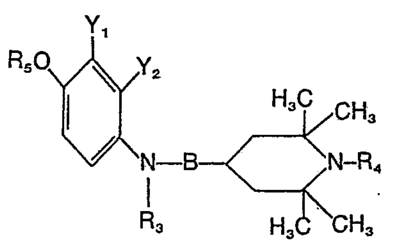

(3)

in welcher $R_5$ ein Wasserstoffatom oder eine Alkyl-$C_1$–$C_4$-, Acetyl-, Propionyl- oder Benzoylgruppe darstellt, und $R_3$, $R_4$, $Y_1$, $Y_2$ und B die weiter oben genannten Bedeutungen haben, in Gegenwart eines dehydratisierenden Kondensationsmittels, wie beispielsweise Schwefelsäure oder Phosphorsäure, bei Temperaturen von etwa 10 bis etwa 200 °C, vorzugsweise etwa 50 bis etwa 150 °C umsetzt. Die Umsetzung nimmt in Abhängigkeit von der angewandten Reaktionstemperatur und den eingesetzten Reaktionskomponenten 1 bis 20 Stunden in Anspruch.

Die Verbindungen der genannten allgemeinen Formeln (2) und (3) können im Molverhältnis 1:1 eingesetzt werden. Es kann jedoch vorteilhaft sein, eine der beiden Komponenten in einem Überschuss bis zu etwa 100 Molprozent einzusetzen.

Nach erfolgter Umsetzung hydrolysiert man das Reaktionsgemisch, in dem man es auf Eiswasser gibt, neutralisiert es dann durch Zugabe einer Base, wie beispielsweise Natronlauge oder Ammoniak, filtriert den abgeschiedenen Feststoff ab, oder extrahiert mit einem mit Wasser nicht mischbaren inerten organischen Lösungsmittel, wie beispielsweise Toluol, und reinigt die angefallene Verbindung der Formel (1), ggfs. nach Abziehen des Lösungsmittels, durch Umkristallisieren in einem geeigneten Lösungsmittel. Zum Umkristallisieren eignen sich beispielsweise Toluol, Halogenkohlenwasserstoffe, Alkohole, aliphatische Kohlenwasserstoffe oder Äther.

Die neuen Verbindungen der genannten Formel (1) können beispielsweise auch hergestellt werden, indem man eine Fluoranverbindung der allgemeinen Formel (4)

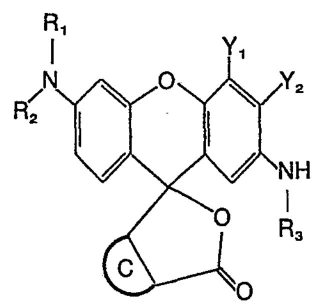

(4)

in welcher $R_1$, $R_2$, $R_3$, $Y_1$, $Y_2$ und C die weiter oben genannten Bedeutungen haben, gegebenenfalls in Gegenwart eines Dehydratisierungsmittels, wie beispielsweise Schwefelsäure oder Phosphorsäure, oder eines Dehydrohalogenierungsmittels, wie beispielsweise einer anorganischen Base, wie Kaliumcarbonat oder Natriumcarbonat, oder einer organischen Base, wie Pyridin oder Triethylamin, mit einer Verbindung der allgemeinen Formel (5)

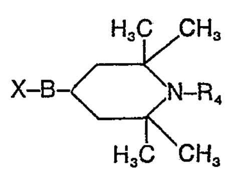

(5)

in welcher X ein Chlor-, Brom- oder Jodatom oder eine Hydroxy-, Acetyl- oder Sulfonylgruppe darstellt und $R_4$ und B die weiter oben genannten Bedeutungen haben, in einem inerten organischen Lösungsmittel, wie beispielsweise Toluol, Cyclohexan oder Dimethylformamid, bei Temperaturen von etwa 20 bis 200 °C, vorzugsweise von etwa 80 bis etwa 150 °C, umsetzt.

Die Verbindungen der genannten allgemeinen Formel (4) und (5) können im Molverhältnis 1:1 eingesetzt werden. Es kann jedoch vorteilhaft sein, eine der beiden Reaktionskomponenten in einem Überschuss bis zu etwa 100 Molprozent anzuwenden.

Nach erfolgter Umsetzung wird das Reaktionsgemisch hydrolysiert, indem man es auf Eiswasser gibt. Anschliessend neutralisiert man durch Zugabe einer Base, wie beispielsweise Natronlauge oder Ammoniak, filtriert den abgeschiedenen Feststoff ab oder extrahiert mit einem mit Wasser nicht mischbaren Lösungsmittel, wie beispielsweise Toluol, und reinigt die angefallene Verbindung der Formel (1), gegebenenfalls nach Abziehen des Lösungsmittels, durch Umkristallisieren in einem geeigneten Lösungsmittel.

Bei den erfindungsgemässen neuen Fluoranverbindungen handelt es sich in der Regel um farblose Verbindungen, die als sogenannte Farbbildner in druck- oder wärmeempfindlichen Aufzeichnungsmaterialien sehr gut geeignet sind. Werden sie mit einer sauren, reaktionsfähigen Substanz, d.h. einem Elektronenakzeptor in Kontakt gebracht, so entwickeln sie mit hoher Geschwindigkeit eine blaue, grüne, rote oder schwarze, die bevorzugten Verbindungen der Formel (2) eine graue bis schwarze Farbe von hoher Farbstärke. Typische Beispiele für die im allgemeinen in den kohlefreien Kopiersystemen verwendeten sauren Coreaktanten sind Bentonite, Attapulgus-Ton, Siliziumdioxid, Aluminiumoxid, Kaolin oder ein sauer reagierendes polymeres Material, wie beispielsweise ein Phenolformaldehydharz. Die neuen Fluoranverbindungen sind in den für die Herstellung von kohlefreien Kopiersystemen üblicherweise verwendeten, nichtflüchtigen Lösungsmitteln farblos und gut löslich und in Wasser unlöslich. Sie lassen sich ausgezeichnet in Mischungen mit anderen Farbbildnern anwenden. Gegenüber den in der DE-OS 2 422 899 und in der DE-OS 2 253 161 beschriebenen Fluoranverbindungen zeichnen sich die erfindungsgemässen tetraalkylpiperidinsubstituierten Fluoranverbindungen durch eine wesentlich bessere Lichtechtheit aus.

In der DE-A-2 121 269 und in der GB-A-1 478 596 werden Fluoranverbindungen ähnlicher Struktur beschrieben, die in 7-Stellung des Fluorans eine Pyridino- oder Methylpyridino-Gruppe enthalten. Die in der DE-A-3 225 028 offenbarten Fluoranverbindungen ähnlicher Struktur enthalten in 7-Stellung des Fluorans über die Iminogruppe als Brükkenglied einen Phenylrest oder eine heterocyclische Gruppe (jedoch keine Pyridinylgruppe). Gegenüber den aus den vorstehend zitierten Literaturstellen bekannten Fluoranverbindungen zeichnen sich die erfindungsgemässen, die ein sterisch stark gehindertes sekundäres oder tertiäres Stickstoffatom enthalten, überraschenderweise durch eine bessere Lichtechtheit aus.

Die neuen Fluoranverbindungen sind als chromogene Elektronendonatoren (Farbbildner) für eine Vielzahl von kohlefreien druckempfindlichen Vervielfältigungssystemen, wie sie beispielsweise in den US-Patentschriften 2 712 507, 2 730 457, 2 932 585, 3 418 250, 3 418 656, 3 427 180, 3 516 846 und 3 672 935 sowie in den britischen Patentschriften 1 042 596, 1 042 599, 1 053 935 und 1 517 650 beschrieben sind, in hervorragender Weise geeignet.

Ferner eignen sie sich auch ausgezeichnet als Farbbildner für thermoreaktive Aufzeichnungsmaterialien, wie sie beispielsweise in der DE-OS 2 110 854 und DE-OS 2 228 581, in der französischen Patentschrift 1 524 826 und in den schweizerischen Patentschriften 407 185, 444 196 und 444 197 beschrieben sind.

Die folgenden Beispiele sollen die Erfindung näher erläutern, ohne sie jedoch darauf zu beschränken. Teile bedeuten in den Beispielen Gewichtsteile.

Beispiel 1

Eine Mischung aus 31,3 Teilen
2-(2′-Hydroxy-4′-diethylamino-benzoyl)-benzoesäure,
27,6 Teilen
4-(1′,2′,2′,6′,6′-Pentamethylpiperidinyl-4′)-aminoanisol
und 150 Teile Schwefelsäuremonohydrat wird 5 Stunden bei 80 °C gerührt und anschliessend auf 1500 Teile Eiswasser gegeben. Nach Neutralisieren mit wässriger Natronlauge wird der entstandene Niederschlag abfiltriert, mit Wasser gewaschen und in einer Toluol/Petrolether-Mischung umkristallisiert.

Man erhält 35 Teile der Verbindung der Formel

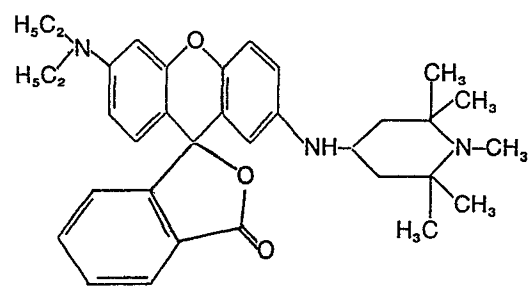

in Form farbloser Kristalle des Schmelzpunktes 153 °C. Eine Lösung dieser Verbindung in Aceton/Wasser (80:20) auf den pH-Wert 3 gestellt, zeigt im UV-Visuellen Spektrum Maxima bei 425, 450 und 586 mm. Eine Lösung der Verbindung in Toluollösung ist farblos und ergibt auf Siliziumoxid eine schwarze, auf Bentonit eine grünstichig schwarze und auf Phenolharz eine dunkelgrüne Farbe.

Beispiel 2

40,0 Teile 2-Amino-3-methyl-6-diethylaminofluoran und 20,4 Teile 1-(2′,2′, 6′,6′-Tetramethylpiperidinyl-4′)-2-chlorethan werden in 50 Teilen N,N-Dimethylformamid unter Zusatz von 10 Teilen Natriumcarbonat 5 Stunden auf 80–90 °C gehalten. Anschliessend wird das Reaktionsgemisch auf Eiswasser gegeben. Die angefallene Mischung wird mit Toluol extrahiert, der Extrakt mit verdünnter wässriger Natronlauge und Wasser gewaschen und mit Natriumsulfat getrocknet.

Beim Eindampfen des Lösungsmittels erhält man die Verbindung der Formel

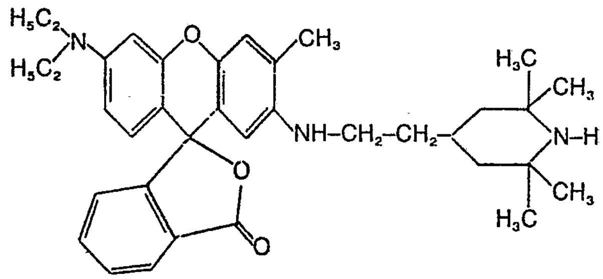

in Form farbloser Kristalle. Die farblose Lösung der Verbindung in Toluol ergibt auf Siliziumdioxid eine schwarze, auf Bentonit eine grünstichig schwarze und auf Phenolharz eine dunkelgrüne Farbe.

Die in der nachstehenden Tabelle aufgeführten neuen Fluoranverbindungen der allgemeinen Formel (1) können nach den weiter oben beschriebenen Verfahren analog den Beispielen 1 und/oder 2 hergestellt werden.

Tabelle

| Bsp. | $R_1 = R_2$ | $R_3$ | $R_4$ | $Y_1$ | $Y_2$ | B | C | Farbton |
|---|---|---|---|---|---|---|---|---|
| 3 | $C_2H_5$ | H | $COCH_3$ | H | H | direkte Bindung | 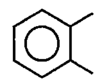 | schwarz |
| 4 | $CH_3$ | $COCH_3$ | $COCH_3$ | H | H | direkte Bindung | 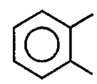 | dunkelrot |
| 5 | 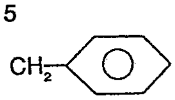 | H | $CH_3$ | H | $CH_3$ | direkte Bindung | 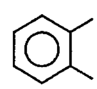 | schwarz |
| 6 | $C_2H_5$ | 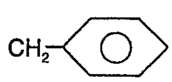 | H | 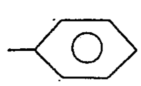 | H | direkte Bindung | 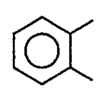 | grün |
| 7 | $C_2H_5$ | $CH_3$ | H | $OC_2H_5$ | H | direkte Bindung | 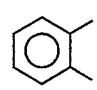 | grün |
| 8 | $C_2H_5$ | $C_2H_5$ | H | H | H | $-CH_2-CH_2-$ | 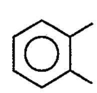 | dunkelgrün |

Tabelle (Fortsetzung)

| Bsp. | $R_1 = R_2$ | $R_3$ | $R_4$ | $Y_1$ | $Y_2$ | B | C | Farbton |
|---|---|---|---|---|---|---|---|---|
| 9 | $CH_3$ | H | $CH_2$- 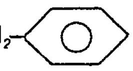 | H | H | $-CH_2-CH_2-$ | 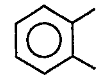 | schwarz |
| 10 | $C_2H_5$ | H | $C_2H_5$ | H | $C_2H_5$ | $-CH_2-CH_2-$ | 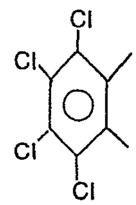 | schwarz |
| 11 | $C_2H_5$ | $CH_3$ | $CH_3$ | Cl | H | $-CH_2-CH_2-$ | 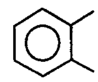 | dunkelgrün |
| 12 | $C_2H_5$ | H | H | H | $OCH_3$ | $-CH_2-CH_2-$ | 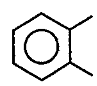 | violett-schwarz |

**Patentansprüche**

1. Fluoranverbindungen der allgemeinen Formel (1)

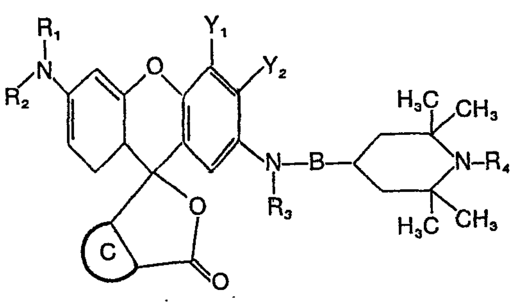 (1)

in welcher $R_1$ und $R_2$ unabhängig voneinander jeweils ein Wasserstoffatom oder eine Alkyl-$C_1$–$C_4$- oder Benzylgruppe, $R_3$ und $R_4$ unabhängig voneinander jeweils ein Wasserstoffatom, eine Alkyl-$C_1$–$C_4$-, Benzyl-, Acetyl-, Propionyl- oder Benzoylgruppe, $Y_1$ und $Y_2$ unabhängig voneinander jeweils ein Wasserstoff-, Chlor- oder Bromatom, Alkyl-$C_1$–$C_4$-, Alkoxy-$C_1$–$C_4$-, Phenyl- oder Naphthylgruppe, B eine direkte Bindung oder die 1,2-Ethylengruppe, und C den ggfs. durch Chlor- oder Bromatome substituierten 1,2-Phenylenrest bedeuten.

2. Fluoranverbindung der Formel

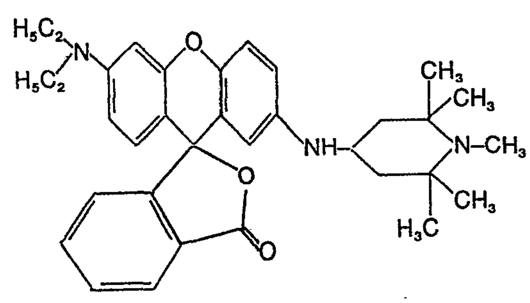

3. Fluoranverbindung der Formel

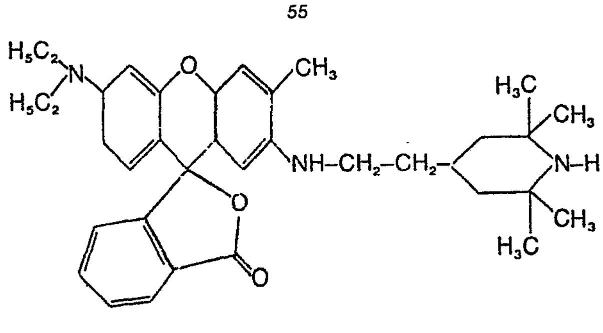

4. Verfahren zur Herstellung von Fluoranverbindungen der in Anspruch 1 genannten allgemeinen Formel (1), dadurch gekennzeichnet, dass man eine Carbonsäure der allgemeinen Formel (2)

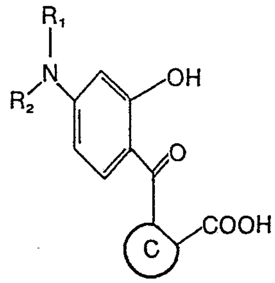

(2)

in welcher $R_1$, $R_2$ und C die in Anspruch 1 genannten Bedeutungen haben, mit einer p-Aminophenolverbindung der allgemeinen Formel (3)

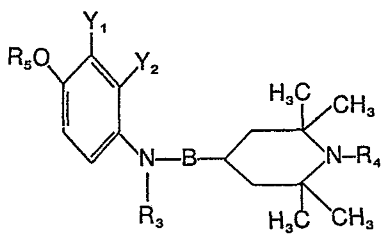

(3)

in welcher $R_5$ ein Wasserstoffatom oder eine Alkyl-$C_1$–$C_4$, Acetyl-, Propionyl- oder Benzoylgruppe darstellt, und $R_3$, $R_4$, $Y_1$, $Y_2$ und B die in Anspruch 1 genannten Bedeutungen haben, in an sich bekannter Weise in Gegenwart eines dehydratisierenden Kondensationsmittels, bei Temperaturen von etwa 10 bis etwa 200 °C umsetzt.

5. Verfahren zur Herstellung von Fluoranverbindungen der in Anspruch 1 genannten allgemeinen Formel (1), dadurch gekennzeichnet, dass man eine Fluoranverbindung der allgemeinen Formel (4)

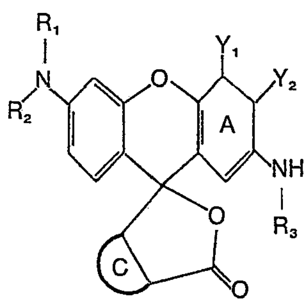

(4)

in welcher $R_1$, $R_2$, $R_3$, $Y_1$, $Y_2$ und C die in Anspruch 1 genannten Bedeutungen haben, ggfs. in Gegenwart eines Dehydratisierungsmittels oder eines Dehydrohalogenierungsmittels mit einer Verbindung der allgemeinen Formel (5)

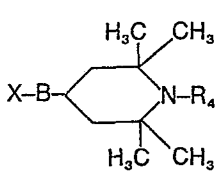

(5)

in welcher X ein Chlor-, Brom- oder Jodatom oder eine Hydroxy-, Acetyl- oder Sulfonylgruppe darstellt und $R_4$ und B die in Anspruch 1 genannten Bedeutungen haben, in bekannter Weise in einem inerten organischen Lösungsmittel bei Temperaturen von etwa 20 bis etwa 200 °C umsetzt.

6. Verwendung der in Ansprüchen 1 bis 4 genannten Fluoranverbindungen als Farbbildner in einem druckempfindlichen oder wärmeempfindlichen Aufzeichnungsmaterial.

**Claims**

1. Fluorane compounds of the general formula (1)

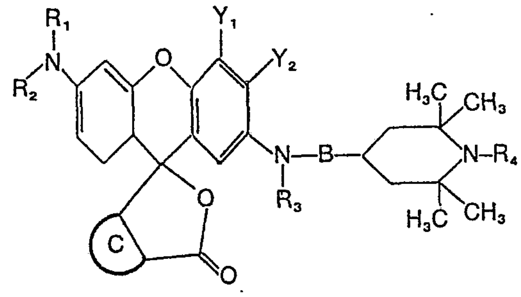

(1)

in which each of $R_1$ and $R_2$ independently of each other denotes a hydrogen atom or an alkyl$C_1$–$C_4$ or benzyl group, each of $R_3$ and $R_4$ independently of each other denotes a hydrogen atom or an alkyl-$C_1$–$C_4$, benzyl, acetyl, propionyl or benzoyl group, each of $Y_1$ and $Y_2$ independently of each other denotes a hydrogen, chlorine or bromine atom or an alkyl$C_1$–$C_4$, alkoxy$C_1$–$C_4$, phenyl or naphthyl group, B denotes a direct bond or the 1,2-ethylene group, and C denotes the optionally chlorine- or bromine-substituted 1,2-phenylene radical.

2. The fluorane compound of the formula

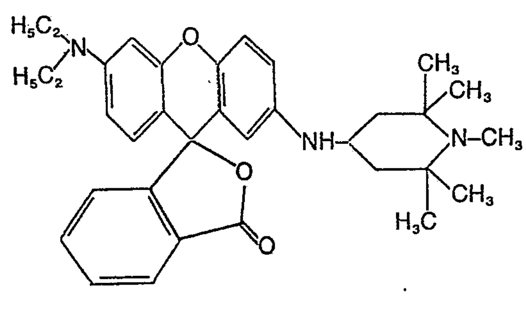

3. The fluorane compound of the formula

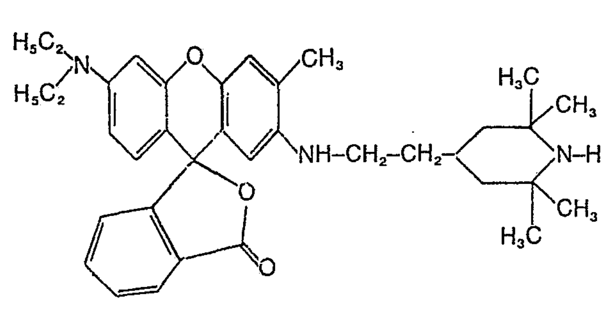

4. A process for preparing fluorane compounds of the formula (1) mentioned in claim 1, which comprises reacting a carboxylic acid of the general formula (2)

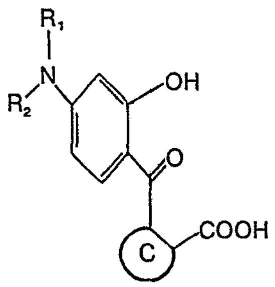

(2)

in which $R_1$, $R_2$ and C have the meanings mentioned in claim 1, with a p-aminophenol compound of the formula (3)

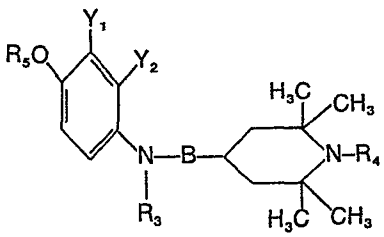

(3)

in which $R_5$ represents a hydrogen atom or an alkyl$C_1$–$C_4$, acetyl, propionyl or benzoyl group, and $R_3$, $R_4$, $Y_1$, $Y_2$ and B have the meanings mentioned in claim 1, in a manner known per se in the presence of a dehydrating condensing agent at temperatures of about 10 to about 200 °C.

5. A process for preparing fluorane compounds of the general formula (1) mentioned in claim 1, which comprises reacting a fluorane compound of the general formula (4)

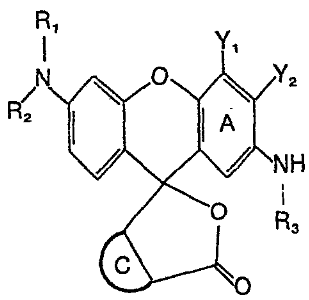

(4)

in which $R_1$, $R_2$, $R_3$, $Y_1$, $Y_2$ and C have the meanings mentioned in claim 1, if appropriate in the presence of a dehydrating agent or of a dehydrohalogenating agent with a compound of the general formula (5)

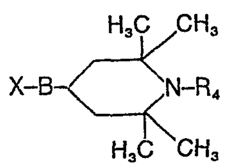

(5)

in which X represents a chlorine, bromine or iodine atom or a hydroxyl, acetyl or sulfonyl group, and $R_4$ and B have the meanings mentioned in claim 1, in a known manner in an inert organic solvent at temperatures of about 20 to 200 °C.

6. The use of fluorane compounds mentioned in claims 1 to 4, as a color former in a pressure-sensitive or heat-sensitive recording material.

**Revendications**

1. Composés de fluorane de formule générale 1 ci-dessous:

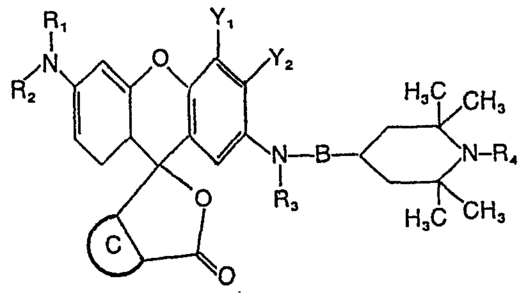

(1)

dans laquelle $R_1$ et $R_2$ représentent chacun, indépendamment l'un de l'autre, un atome d'hydrogène, un alkyle en $C_1$–$C_4$ ou un groupe benzyle, $R_3$ et $R_4$ représentent chacun, indépendamment l'un de l'autre, un atome d'hydrogène, un alkyle en $C_1$–$C_4$ ou un groupe benzyle, acétyle, propionyle ou benzoyle, $Y_1$ et $Y_2$ représentent chacun, indépendamment l'un de l'autre, un atome d'hydrogène, de chlore ou de brome, un alkyle en $C_1$–$C_4$, un alcoxy en $C_1$–$C_4$ ou le groupe phényle ou naphtyle, B désigne une liaison directe ou le groupe 1,2-éthylène et C le radical 1,2-phénylène portant éventuellement des atomes de chlore ou de brome.

2. Le composé de fluorane de formule:

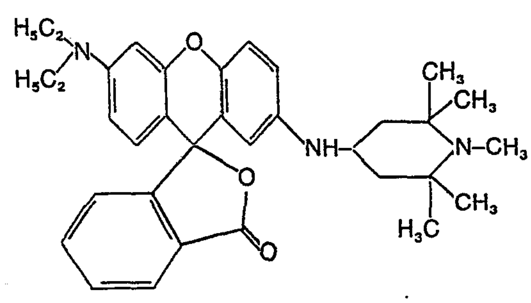

3. Le composé de fluorane:

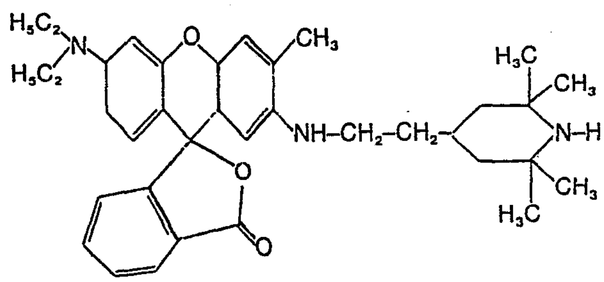

4. Procédé de préparation de composés de fluorane de formule 1 selon la revendication 1,

procédé caractérisé en ce que l'on fait réagir un acide carboxylique de formule générale 2:

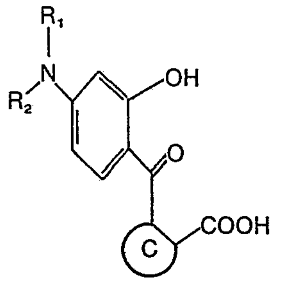

(2)

avec un composé p-aminophénolique de formule 3:

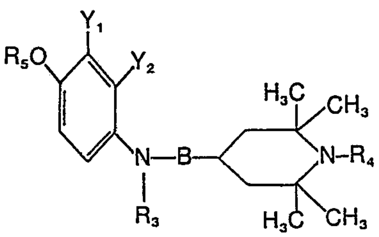

(3)

$R_5$ désignant un atome d'hydrogène, un alkyle en $C_1$–$C_4$ ou le groupe acétyle, propionyle ou benzoyle et les autres symboles ayant les mêmes significations que dans la revendication 1, de manière connue, en présence d'un agent de condensation déshydratant, à des températures d'environ 10 à 200 °C.

5. Procédé de préparation de composés de fluorane de formule 1 selon la revendication 1, procédé caractérisé en ce que l'on fait réagir un composé du fluorane de formule générale 4:

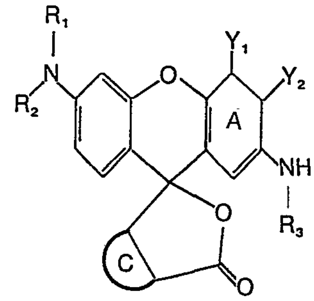

(4)

éventuellement en présence d'un agent déshydratant ou d'un agent de déshydrohalogénation, avec un composé de formule générale 5:

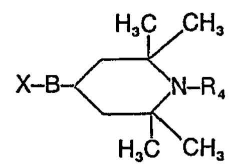

(5)

dans laquelle X désigne un atome de chlore, de brome ou d'iode ou un groupe hydroxyle, acétyle ou sulfonyle, de manière connue, dans un solvant organique inerte à des températures d'environ 20 à 200 °C.

6. L'utilisation des composés de fluorane selon les revendications 1 à 3 comme formateurs de couleurs dans une matière d'enregistrement sensible à la pression ou à la chaleur.